(19) Europäisches Patentamt
European Patent Office
Office européen des brevets

(11) **EP 4 342 515 B1**

(12) # EUROPEAN PATENT SPECIFICATION

(45) Date of publication and mention
of the grant of the patent:
**02.07.2025   Bulletin 2025/27**

(21) Application number: **23199007.8**

(22) Date of filing: **22.09.2023**

(51) International Patent Classification (IPC):
***A61N 1/32*** *(2006.01)*

(52) Cooperative Patent Classification (CPC):
**A61N 1/327**

---

(54) **IMPROVED ELECTROPORATION DEVICE**

VERBESSERTE ELEKTROPORATIONSVORRICHTUNG

DISPOSITIF D'ÉLECTROPORATION AMÉLIORÉ

---

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **23.09.2022  IT 202200019635**

(43) Date of publication of application:
**27.03.2024   Bulletin 2024/13**

(73) Proprietor: **IGEA S.p.A.**
**41012 Carpi (MO) (IT)**

(72) Inventors:
• **FUSCO, Roberta**
**41012 CARPI (MO) (IT)**

• **DE TERLIZZI, Francesca**
**41012 CARPI (MO) (IT)**
• **CADOSSI, Ruggero**
**41012 CARPI (MO) (IT)**
• **MARAZZI, Donata**
**41012 CARPI (MO) (IT)**

(74) Representative: **Studio Torta S.p.A.**
**Via Viotti, 9**
**10121 Torino (IT)**

(56) References cited:
WO-A1-2020/061192     US-A1- 2019 336 757
US-A1- 2020 197 073     US-A1- 2021 023 362
US-B1- 10 814 129       US-B2- 7 053 063

---

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the art**

The present invention relates to an electroporation device.

**Background art**

As is well known, in many surgical and ablative treatments (e.g. aimed at removing malignant or benign tumours), it is important to remove a specific pathological tissue without causing or minimising damage to the surrounding healthy tissue that does not need to be removed.

Electroporation treatments have recently been developed in this field, which are designed to eliminate specific portions of target cell tissue while leaving the surrounding portions unaffected.

As is well known, electroporation treatments involve the application of electric pulses to a tissue by means of electrodes applied to the tissue itself; the electric field generated produces the formation of pores in the cell membrane, causing a change in the cell membrane that eases the flow of organic/inorganic substances (e.g. DNA or drugs) from outside to inside the cell.

These electroporation treatments may be controlled according to the parameters (voltage, waveform, duty cycle, application time, number of applied pulses, etc.) of the electric pulses; by varying these parameters, a reversible or irreversible electroporation can be obtained.

In the first case, the pores of the cell membrane close up following the cessation of pulses and the cells continue to live. In the second case, the high electric field applied to the cells produces pores of such a size that the integrity of the cell membrane cannot be reconstructed and results in cell lysis. Modest thermal effects are present at the surface of the electrodes.

Document WO 2005065284 by DAVALOS, Rafael and RUBINSKY, Boris details the limitations of the electroporation process.

In particular, it is noted how, by controlling the intensity of the applied field and the amplitude of the pulses, it is possible to obtain:

- no effect;
- a reversible-type electroporation; or
- an irreversible electroporation causing cell lysis.

According to the above-mentioned document, irreversible electroporation is obtained by applying pulses with a duration of at least 100 microseconds and a sufficiently high amplitude to induce irreversible damage to the cell membrane causing the death of the cell itself. Reversible pore formation is not expected to be accompanied by lysis or cell death. In case of electric pulses with a high amplitude and sufficient duration, cell lysis is observed. The application of such pulses is accompanied by a high electron flow that causes a state of diffuse inflammation in the tissue.

The object of the present invention is to create a reversible electroporation device adapted to promote the diffusion and passage of drugs or organic molecules (DNA and RNA) .

Other earlier documents are: US 7,053,063, WO2020/061,192, US 2021/023,362, US 2019/336,757, US 10,814,129 and US 2020/197,073.

**Summary of the invention**

The preceding object is achieved by the present invention in that it relates to an electroporation device made according to claim 1.

**Brief description of the drawings**

The invention will now be illustrated with reference to the accompanying drawings wherein:

Figure 1 schematically shows an electroporation device made according to the dictates of the present invention;
Figure 2 shows a block diagram of the device operations according to the present invention; and
Figure 3 shows some experimental results obtained with the device in Figure 1.

## Description of the embodiment

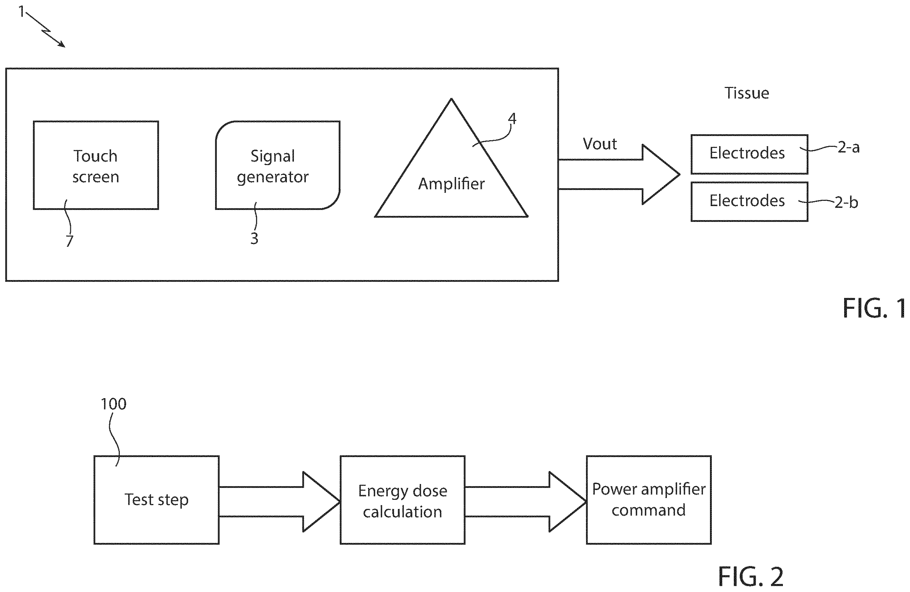

**In Figure 1,** an electroporation device which is adapted to provide at least one pair of electrodes 2-a, 2-b with a periodic voltage $V_{out}$ having an amplitude and duration which can be controlled as shown hereinafter, is globally referred to as 1. In the non-limiting example shown, a pair of electrodes is present, however, it goes without saying that a different number of electrodes can be used, e.g. four, six or a bipolar electrode.

The electrodes 2-a, 2-b with an elongated rectilinear shape have a length **1,** a section **S** and are arranged facing each other and spaced by a distance **d** approximately corresponding to the thickness of the area to be treated.

The electroporation device 1 comprises an electronic unit provided with a signal generator 3 that supervises signal generation and a power amplifier 4 that supplies the voltage $V_{out}$ to the electrodes 2-a, 2-b.

In use, the pair of electrodes 2-a, 2-b is inserted into a portion of tissue T of a human body to be treated (schematically shown).

The operations performed by the device 1 are the following ones.

## Block 100 (test step)

The electronic unit commands the following operations.

An initial test pulse (also called "pre-pulse") **$V_{test}$** is generated and supplied to the pair of electrodes 2-a, 2-b and therefore to the tissue T;

Measure the current $I_{test}$ flowing between the pair of electrodes 2-a, 2-b (the current depends on the characteristics of the tissue T);

Detect the resistance value $R_{A,B}$ between the pair of electrodes according to Ohm's law as:

$$R_{A,B} = (V_{test}) / (I_{test})$$

Store the resistance value $R_{A,B}$ detected.

Calculate, according to the resistance value calculated $R_{A,B}$ and according to a maximum current **$I_{MAX}$** that can be supplied by the power amplifier 4, the maximum voltage deliverable $V_{MAX}$ as:

$$V_{MAX} = R_{A,B} \times I_{MAX}$$

The maximum current supplied by the electroporation device **$I_{MAX}$** is a function of the technical characteristics of the power amplifier 4 of the electroporation device and it is a known datum, e.g. **$I_{MAX}$** = **50** amperes.

As a result of the above mentioned operations, the electroporation device operates with the following constraint V < **$V_{MAX}$**, i.e. it generates a voltage limited to **$V_{MAX}$** such that the current supplied to the electrodes is always less than the maximum deliverable current.

## Block 110 energy dose calculation.

Next, the value of the spacing d between the electrodes 2-a, 2-b is supplied to the device 1.

The distance d is supplied by the operator using the electroporation device, e.g. by a command given via touch screen 7 or keyboard.

The block 110 calculates the total <u>duration</u> **$\tau(n,t)$** (expressed in seconds) of exposure to the local electric field of an electroporation signal comprising several pulses of fixed duration and <u>the local electric field intensity</u> **E(k** ) (expressed in volts per cm) that must be supplied to such a tissue T in order to administer to such tissue T an Absorbed Dose (AD, expressed in J/g) that is **greater than** the energy dose $AD_R$ that achieves a reversible electroporation. The energy dose $AD_R$ is a known value stored in the device 1 and is derived experimentally through a series of tests. The threshold value of absorbed energy must be exceeded over the entire target volume to be electroporated.

The total duration of exposure to the local electric field $\tau(n,t)$, in the case of periodic pulses (e.g. a square wave), is given by the number n of pulses multiplied by the time duration t of each pulse.

The local electric field intensity E(k), on the other hand, is given by the voltage V applied between two electrodes 2-a, 2-b spaced by the distance d (expressed in cm), i.e. E(k) = V/d.

In particular, the absorbed energy dose AD from the tissue T can be derived according to the following formula:

$$AD = \frac{\sigma \cdot [E(k)]^2 \cdot \tau(n,t)}{\rho}$$

Where σ represents the conductivity of the tissue (in Siemens/cm),
And the local electric field intensity (in V/cm),
τ the total duration of the exposure to the local electric field (in sec),
ρ the density of the tissue (as g/cm$^3$).

The absorbed dose AD is expressed in J/g.

σ and ρ are known as properties of the tissue T undergoing electroporation.

In particular:

σ is defined as the inverse of resistivity ϱ i.e. σ =1/ϱ.

The resistivity ϱ = R*S/d where R denotes the electrical resistance and S the section of the electrodes.

Thus, starting from the resistance calculated with the pre-pulse $R_{A,B}$ and knowing the distance d between the two electrodes and the section of the electrodes 2-a, 2-b we derive σ = d / (R*S).

The electric field E is a function of the voltage V and the distance d between the electrodes 2-a, 2-b i.e. E = V/d. The formula can therefore be expressed as:

$$AD = \frac{\sigma \cdot [(V/\ d)]^2 \cdot \tau(n,t)}{\rho}$$

The electronic unit calculates and selects a pair of values of total <u>duration</u> τ(n,t) and voltage V that satisfy the inequality:

$$\frac{\sigma \cdot [(V/\ d)]^2 \cdot \tau(n, t)}{\rho} > AD_R$$

Where as stated above:

$AD_R$ represents the energy dose that performs a reversible electroporation - The energy dose $AD_R$ is a known value stored in the device 1 and is derived experimentally.

σ is defined as the inverse of resistivity σ =1/ϱ;

the resistivity is calculated from the electronic unit as ϱ = R*S/d where R denotes the electrical resistance (measured in block 100), d the distance between the electrodes (known datum) and S the section (known datum) of the electrodes 2-a, 2-b;

The selection of the voltage torque V and total <u>duration</u> is realised by iterative methods, i.e. by randomly fixing one of the two parameters V and τ(n,t) by finding the other parameter in such a way that the inequality is satisfied. As far as the voltage is concerned, however, the constraint according to which it is below Vmax and above a threshold value is maintained, hence no electroporation effects are obtained.

For example, applying a voltage of V=1000 V and a duration of τ=800 μsec (i.e. 8 pulses with a duration of 100 psec), an energy dose is obtained which can also be obtained by applying a voltage of 700 V with a duration of 4000 μsec, or by applying a voltage of 500 V with a duration of 8000 μsec. Block 120 is followed by block 130, which commands the power amplifier for generating the voltage thus determined V and applying this voltage to the electrodes for the determined time τ(n,t).

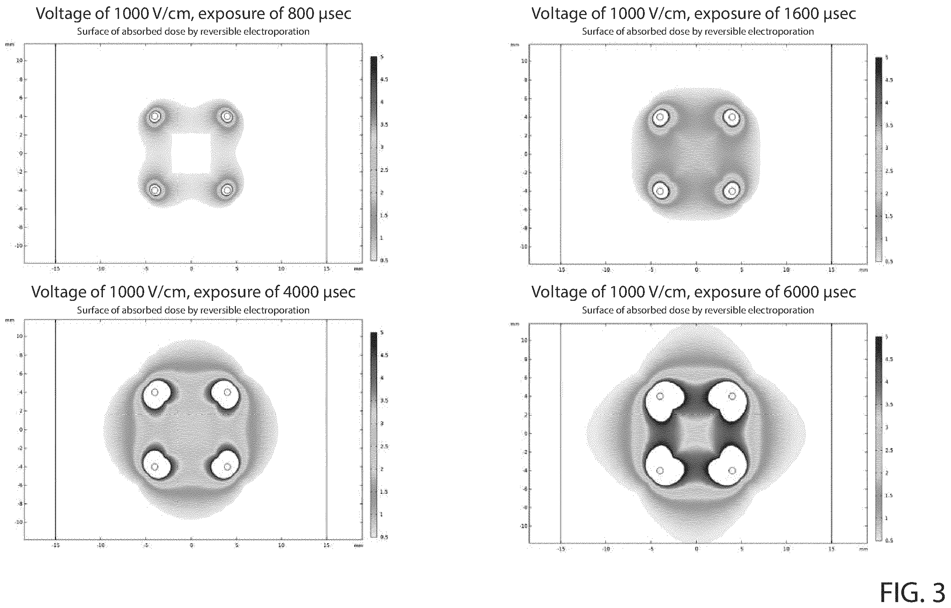

Figure 3 shows the areas of reversible electroporation obtained from four electrodes with an applied voltage of 1000 V/cm and different values of the total duration τ of exposure to the local electric field.

The size of the electroporated areas in the 2D model are shown in Table 1.

Table 1. Reversible electroporation area as the exposure time to the local electric field changes.

| Applied potential | Exposure time | Electroporated area |
|---|---|---|
| 1000 V | 800 μsec | 100.5 mm$^2$ |
| 1000 V | 1600 μsec | 172.1 mm$^2$ |
| 1000 V | 4000 μsec | 265.8 mm$^2$ |
| 1000 V | 6000 μsec | 328.6 mm$^2$ |

**Claims**

1.  An electroporation device adapted to supply to at least a pair of electrodes (2-a, 2-b) available in a portion of tissue (T) of the human body a periodic voltage $V_{out}$ having voltage V and controllable total duration $\tau$;

    the electrodes (2-a, 2-b) with elongated rectilinear shape have a section **s** and are arranged facing and spaced from each other by a distance **d;**
    the electroporation device (1) comprises an electronic unit provided with a signal generator (3) and a power amplifier (4) which supplies the voltage $V_{out}$ to the electrodes (2-a, 2-b);

    the electroporation device (1) is configured to perform the following operations:

    generate at least a first test pulse $\mathbf{V_{test}}$ which is supplied to the pair of electrodes (2-a, 2-b) and therefore to the tissue T;
    measure the current $I_{test}$ which flows between the pair of electrodes (2-a, 2-b) and which depends on the tissue characteristics (T);
    detect the resistance value $R_{A,B}$ between the pair of electrodes according to Ohm's law as:

    $$R_{A,B} = (V_{test}) / (I_{test})$$

    store the resistance value $\mathbf{R_{A,B}}$ detected;
    **characterized by** calculating, selecting and storing a pair of values of total <u>duration</u> $\tau$ (n,t) and voltage V that satisfy the inequality:

    $$\frac{\sigma \cdot [(V/d)]^2 \cdot \tau(n,t)}{\rho} > AD_R$$

    where:

    $\mathbf{AD_R}$ represents the energy dose that realises a reversible electroporation - the energy dose $AD_R$ is a known value stored in the device (1);

    $\sigma$ is defined as the inverse of resistivity $\sigma = 1/\varrho$;
    d is the spacing of the electrodes;
    $\tau$(n,t) represents the total duration of exposure to the local electric field in the case of periodic pulses that is given by the number n of pulses multiplied by the time duration t of each pulse;

    the resistivity $\varrho$ = R*S/d where R denotes the measured and stored resistance value and S the section of the electrodes (2-a, 2-b); and control the power amplifier (4) so that a voltage equal to the voltage V is applied to the electrodes and supplied to the tissue portion for the determined total duration $\tau$(n,t).

2.  The device according to claim 1 wherein selection of the voltage torque V and total <u>duration</u> is obtained by means of iterative methods, namely by fixing at random one of the two parameters V and $\tau$ (n,t), finding the other parameter so that the inequality is satisfied.

3.  The device according to claim 2 wherein the electroporation device (1) is furthermore configured to carry out the following operations:
    calculate, according to the resistance value calculated $R_{A,B}$ and according to a maximum current $\mathbf{I_{MAX}}$ that can be supplied by the power amplifier (4), the maximum voltage deliverable $V_{MAX}$ as:

    $$\mathbf{V_{MAX} = R_{A,B} \times I_{MAX}}$$

    the maximum current supplied by the electroporation device $\mathbf{I_{MAX}}$ is a function of the technical characteristics of the power amplifier (4) of the electroporation device and is a known datum;
    limit the voltage delivered to a value below $\mathbf{V_{MAX}}$ so that the current supplied to the electrodes is below the maximum deliverable current.

4.  The device according to claim 3 wherein selection of the voltage torque V and total <u>duration</u> is achieved by selecting a voltage below $V_{MAX}$ and above a threshold value whereby electroporation effects are not obtained.

**Patentansprüche**

1. Elektroporationsvorrichtung, die dazu ausgelegt ist, mindestens einem Paar von Elektroden (2-a, 2-b), die in einem Abschnitt des Gewebes (T) des menschlichen Körpers verfügbar sind, eine periodische Spannung $V_{out}$ mit der Spannung V und einer steuerbaren Gesamtdauer $\tau$ zuzuführen;

   die Elektroden (2-a, 2-b) mit länglicher geradliniger Form einen Querschnitt **S** aufweisen und einander zugewandt und um einen Abstand **d** voneinander beabstandet angeordnet sind;
   die Elektroporationsvorrichtung (1) eine elektronische Einheit umfasst, die mit einem Signalgenerator (3) und einem Leistungsverstärker (4) versehen ist, der den Elektroden (2-a, 2-b) die Spannung $V_{out}$ zuführt;
   die Elektroporationsvorrichtung (1) ist so konfiguriert, dass sie die folgenden Vorgänge durchführt:

   Erzeugen mindestens eines ersten Testimpulses $V_{test}$, der dem Paar von Elektroden (2-a, 2-b) und daher dem Gewebe T zugeführt wird;
   Messen des Stroms $I_{test}$, der zwischen dem Paar von Elektroden (2-a, 2-b) fließt und der von den Gewebeeigenschaften (T) abhängt;
   Detektieren des Widerstandswerts $R_{A,B}$ zwischen dem Paar von Elektroden, gemäß dem Ohmschen Gesetz als:

   $$R_{A,B} = (V_{test}) \; / \; (I_{test})$$

   Speichern des detektierten Widerstandswert $R_{A,B}$;
   **gekennzeichnet durch** Berechnen, Auswählen und Speichern eines Paars von Werten der Gesamt<u>dauer</u> $\tau$ (n,t) und Spannung V, die die Ungleichung erfüllen:

   $$\frac{\sigma \cdot [(V/d)]^2 \cdot \tau(n,t)}{\rho} > AD_R$$

   WO:

   $AD_R$ die Energiedosis darstellt, die eine reversible Elektroporation bewirkt - die Energiedosis $AD_R$ ein bekannter Wert ist, der in der Vorrichtung (1) gespeichert ist;

   $\sigma$ definiert als der Kehrwert des spezifischen Widerstands $\sigma = 1/\varrho$ ist;
   d der Abstand der Elektroden ist;
   $\tau$(n,t) die Gesamtdauer der Exposition gegenüber dem lokalen elektrischen Feld bei periodischen Impulsen darstellt, die durch die Anzahl n von Impulsen multipliziert mit der Zeitdauer t jedes Impulses gegeben ist;

   den spezifischen Widerstand $\varrho$ = R*S/d, wobei R den gemessenen und gespeicherten Widerstandswert und S den Querschnitt der Elektroden (2-a, 2-b) bezeichnet; und den Leistungsverstärker (4) so zu steuern, dass eine Spannung gleich der Spannung V an die Elektroden angelegt und dem Gewebeabschnitt für die bestimmte Gesamtdauer $\tau$(n,t) zugeführt wird.

2. Vorrichtung nach Anspruch 1, wobei die Auswahl des Spannungsdrehmoments V und der Gesamt<u>dauer</u> mittels iterativer Verfahren erhalten wird, nämlich durch zufälliges Festlegen eines der zwei Parameter V und $\tau$ (n,t), Auffinden des anderen Parameters, so dass die Ungleichung erfüllt ist.

3. Vorrichtung nach Anspruch 2, wobei die Elektroporationsvorrichtung (1) ferner so konfiguriert ist, dass sie die folgenden Vorgänge durchführt:
   Berechnen, entsprechend dem berechneten Widerstandswert $R_{A,B}$ und entsprechend einem maximalen Strom $I_{MAX}$, der von dem Leistungsverstärker (4) zugeführt werden kann, der maximal lieferbaren Spannung $V_{MAX}$:

   $$V_{MAX} = R_{A,B} \; x \; I_{MAX}$$

der von der Elektroporationsvorrichtung zugeführte maximale Strom $I_{MAX}$ ist eine Funktion der technischen Eigenschaften des Leistungsverstärkers (4) der Elektroporationsvorrichtung und ein bekanntes Datum ist; Begrenzen der gelieferten Spannung auf einen Wert unter $V_{MAX}$, damit der den Elektroden zugeführte Strom unter dem maximal lieferbaren Strom liegt.

4. Vorrichtung nach Anspruch 3, wobei das Auswählen des Spannungsdrehmoments V und der Gesamtdauer durch Auswählen einer Spannung unterhalb von $V_{MAX}$ und oberhalb eines Schwellenwerts erreicht wird, wodurch keine Elektroporationseffekte erzielt werden.

**Revendications**

1. Dispositif d'électroporation adapté pour fournir à au moins une paire d'électrodes (2-a, 2-b) disponibles dans une partie de tissu (T) du corps humain une tension périodique $V_{out}$ ayant une tension V et une durée totale contrôlable $\tau$ ;

les électrodes (2-a, 2-b) de forme rectiligne allongée ont une section **S** et sont disposées face à face et espacées les unes des autres d'une distance **d** ;
le dispositif d'électroporation (1) comprend une unité électronique dotée d'un générateur de signaux (3) et d'un amplificateur de puissance (4) qui fournit la tension $V_{out}$ aux électrodes (2-a, 2-b) ;
le dispositif d'électroporation (1) est configuré pour effectuer les opérations suivantes :

la génération d'au moins une première impulsion de test $V_{test}$ qui est fournie à la paire d'électrodes (2-a, 2-b) et donc au tissu T ;
la mesure du courant $I_{test}$ qui circule entre la paire d'électrodes (2-a, 2-b) et qui dépend des caractéristiques du tissu (T) ;
la détection de la valeur de la résistance $R_{A,B}$ entre la paire d'électrodes selon la loi d'Ohm comme suit :

$$R_{A,B} = (V_{test}) / (I_{test})$$

la mémoire de la valeur de résistance $R_{A,B}$ détectée ;
**caractérisé par** le calcul, la sélection et le stockage d'une paire de valeurs de durée totale $\tau$ (n,t) et de tension V qui satisfont à l'inégalité :

$$\frac{\sigma \cdot [(V/d)]^2 \cdot \tau(n,t)}{\rho} > AD_R$$

où :

$AD_R$ représente la dose d'énergie qui réalise une électroporation réversible - la dose d'énergie $AD_R$ est une valeur connue stockée dans le dispositif (1) ;
$\sigma$ est définie comme l'inverse de la résistivité $\sigma = 1/\varrho$ ;
d est l'espacement des électrodes ;
$\tau(n,t)$ représente la durée totale d'exposition au champ électrique local dans le cas d'impulsions périodiques, qui est donnée par le nombre n d'impulsions multiplié par la durée t de chaque impulsion ;
la résistivité $\varrho = R*S/d$ où R représente la valeur de la résistance mesurée et stockée et S la section des électrodes (2-a, 2-b) ; et la commande de l'amplificateur de puissance (4) de sorte qu'une tension égale à la tension V soit appliquée aux électrodes et fournie à la partie de tissu pendant la durée totale déterminée $\tau(n,t)$.

2. Dispositif selon la revendication 1 dans lequel la sélection du couple de tension V et de la durée totale est obtenue au moyen de méthodes itératives, à savoir en fixant au hasard l'un des deux paramètres V et $\tau$ (n,t), en trouvant l'autre paramètre de manière à ce que l'inégalité soit satisfaite.

3. Dispositif selon la revendication 2, dans lequel le dispositif d'électroporation (1) est en outre configuré pour effectuer les opérations suivantes :
le calcul, en fonction de la valeur de résistance calculée $R_{A,B}$ et en fonction d'un courant maximal $I_{MAX}$ pouvant être fourni par l'amplificateur de puissance (4), de la tension maximale délivrable $V_{MAX}$ comme suit :

$$V_{MAX} = R_{A,B} \times I_{MAX}$$

le courant maximal fourni par le dispositif d'électroporation $I_{MAX}$ est une fonction des caractéristiques techniques de l'amplificateur de puissance (4) du dispositif d'électroporation et est une donnée connue ;
la limite de la tension délivrée à une valeur inférieure à $V_{MAX}$ afin que le courant fourni aux électrodes soit inférieur au courant maximal délivrable.

4. Dispositif selon la revendication 3 dans lequel la sélection du couple de tension V et de la durée totale est réalisée en sélectionnant une tension inférieure à $V_{MAX}$ et supérieure à une valeur seuil par laquelle les effets d'électroporation ne sont pas obtenus.

EP 4 342 515 B1

FIG. 1

FIG. 2

Voltage of 1000 V/cm, exposure of 800 μsec
Surface of absorbed dose by reversible electroporation

Voltage of 1000 V/cm, exposure of 1600 μsec
Surface of absorbed dose by reversible electroporation

Voltage of 1000 V/cm, exposure of 4000 μsec
Surface of absorbed dose by reversible electroporation

Voltage of 1000 V/cm, exposure of 6000 μsec
Surface of absorbed dose by reversible electroporation

FIG. 3

**REFERENCES CITED IN THE DESCRIPTION**

**Patent documents cited in the description**

- WO 2005065284 A, DAVALOS, Rafael and RUBINSKY, Boris
- US 7053063 B
- WO 2020061192 A
- US 2021023362 A
- US 2019336757 A
- US 10814129 B
- US 2020197073 A